# EUROPEAN PATENT APPLICATION

(11) **EP 1 172 753 A2**
(43) Date of publication of application: **16.01.2002**
(21) Application number: 01306037.1
(22) Date of filing: 13.07.2001
(51) Int. Cl.: G06F 19/00

(54) **Method for optimising pharmatheutical prescribing**

(30) Priority: 15.07.2000 GB 0173187; 22.09.2000 GB 0232777
(71) Applicant: Bodsworth, Andrew William, Northampton NN5 6NR (GB); Ackford, Kelvin, Kislingbury, Northamptonshire (GB); Berryman, Matthew Oliver, Pennsylvania, Exeter EX4 5HJ (GB)
(72) Inventor: Bodsworth, Andrew William, Northampton NN5 6NR (GB); Ackford, Kelvin, Kislingbury, Northamptonshire (GB); Berryman, Matthew Oliver, Pennsylvania, Exeter EX4 5HJ (GB)
(74) Representative: Gregory, Timothy Mark

(57) **Abstract**

The method permits alerting a medical practitioner to information regarding up-to-date and cost-effective alternatives to a medical prescription. A proposed prescription is compared to a database of prescriptions and possible alternative prescriptions which could be efficacious. If there is a suitable alternative or alternatives corresponding to the prescription, it is shown to the practitioner. The practitioner may then choose between his proposed prescription and the alternative or alternatives presented to him. The practitioner may then enter either the proposed or an alternative prescription to a system adapted to process and print out prescriptions.

## Description

The present invention relates to the field of computer-based systems to support the activities of a medical practitioner. More particularly, it relates to a computer-based system for minimising the cost of prescribing whilst maintaining therapeutic effectiveness, utilising the most recent available data.

Medical practitioners have a range of computer-based systems at their disposal to lessen the burden of administrative aspects of their activities. In addition to the use of general business management packages, which are also applicable to the operation of a medical practice, computer systems are routinely entrusted with the storage and recovery of patients' details and medical histories, and with printing out prescriptions in standardised formats. Certain medical volumes and references are available in CD-ROM and other formats and so can be consulted on screen.

However, such is the progress of medical science, particularly in the pharmaceutical field, that a medical practitioner cannot be expected to keep up to date with every development. These developments include not only new, more effective drugs, preparations and methods of administration, but also such matters as the availability of "generic" equivalents to well known branded pharmaceuticals, the availability of alternative sources of medical materials at lower prices, the optimisation of the dosage taken by the patient, and all such information relating to available alternative formats.

The National Health Service or equivalent authority employs "prescribing leads", providing pamphlets which describe which drugs are to be prescribed in preference to others, for certain conditions. The time taken to develop, produce, print and distribute these pamphlets is such that they may be out of date before they are delivered.

The sheer volume of information which needs to be delivered also makes such a means of delivery impracticable.

There is also no guarantee that a medical practitioner will have the time to read and absorb this much information, such that he or she will recall it when needed.

As a result, there is a "knowledge deficit" between the sum total of available pharmacological information, which includes the uses, dosages, formulations, strengths and costs of pharmaceuticals, and medical practitioners' prescribing habits. It is estimated that such "inefficient" prescribing by general practitioners may cost around £600 million per year in the UK alone. While medical practitioners are fully cognisant of the need to keep up with clinical data, they are not predisposed, either by training or by workload, to take into account the relative costs of alternative prescriptions, even when there is no clinical benefit from the more expensive option.

There are many alternative options, or "switches", which could be considered without in any way encroaching on a medical practitioner's professional judgement or clinical effectiveness. These include, by way of example, but not exclusively:-
- the substitution of "generic" equivalents for materials no longer subject to patent protection;
- the use of alternative drugs or preparations with a lower cost per effective dose; and
- the use of different strengths, dose sizes and/or frequencies of administration to optimise the drug cost and therapeutic benefit.
- the substitution of a drug without a particular side-effect for a combination of one drug with that side-effect, plus a further drug to treat the side-effect.

If information concerning such matters could be brought to the attention of a medical practitioner when he actually needs it, he would be more inclined to consider such factors when making his prescribing decisions.

It is therefore an object of the present invention to provide a method of alerting a medical practitioner to up-to-date information as may be relevant, regarding alternatives to a prescription, at a point at which said information would be of greatest use.

According to a first aspect of the present invention, there is provided a system to supply a medical practitioner with possible alternatives to a proposed prescription, characterised in that it comprises means to compare said prescription to a database of prescriptions, means to select from said database the proposed prescription and any possible alternative thereto, means to present an alternative prescription to the practitioner, whereby the practitioner may choose between his proposed prescription and any alternative presented to him, means to permit the practitioner to enter a prescription selected from the proposed or an alternative prescription, and means to process and print out the selected prescription.

Preferably, the system is provided with a means of updating said database of prescriptions and possible alternatives, optionally with data provided by a central source.

Advantageously, updates to said database are provided online, by means of electronic mail or the Internet, in which case said database may be updated either on a real-time basis, as new information becomes available to the central source, or periodically, for example, daily, hourly or every ten minutes.

Alternatively, said updates may be provided by CD-ROM, by floppy disc or by other suitable pre-programmed means according to available resources.

In a preferred embodiment of the present invention, the alternatives to the proposed prescription are provided to the practitioner along with further medical or cost information to explain or expand on the reasons for considering said alternatives.

Advantageously, means are provided to enable the practitioner to access more detailed information if he so wishes, which more detailed information may be provided as part of said database or, optionally, may be held at said central source, in which case it may be provided by, for example, arranging access to restricted access websites.

Preferably, the system is provided with means to supply, and optionally to print out, explanatory information adapted for the benefit of a patient for whom the prescription is intended.

In another preferred embodiment of the present invention, said database of prescriptions and possible alternatives also comprises data on costs of the prescriptions and of the alternatives, and the system is provided with means to rank said alternatives on the basis of cost and means to present said alternatives to the practitioner along with an indication of the costs involved.

Advantageously, the system may also be provided with means to calculate any savings made when the practitioner does choose an alternative prescription, and to record said savings in a further database, optionally linked to data such as, for example, but not exclusively, the prescription replaced, the alternative chosen and the practitioner making the choice.

An operator may then analyse said further database to establish and report on which alternatives or "switches" are being used most often, or which are producing the greatest cost savings, optionally compared with the savings expected when the alternatives were introduced.

The system may transfer such data automatically to a central point, for example, the central source of data on alternative prescriptions, to enable such analysis to be carried out on combined data from several medical practices.

Further management information may be collected, analysed and reported, preferably in a format not identifying individual practitioners, to aid efficient administration of this aspect of medical practice.

According to a second aspect of the present invention, there is provided a method of supplying a medical practitioner with possible alternatives to a proposed prescription, which responds to an input of said prescription to a first computer program adapted to process and print out said prescription characterised in that the method comprises the steps of:-
- interrupting operation of said first program;
- comparing said prescription to a database of prescriptions and possible alternative prescriptions thereto;
- presenting any prescriptions alternative to said input prescription to the practitioner;
- providing the practitioner with means to choose between said input prescription and any alternative presented to him; and
- resuming operation of said first program, either using original data corresponding to the input prescription or using substitute data supplied by the system corresponding to a selected alternative presented, according to the choice made by the practitioner.

Preferably, the system is adapted to supply said substitute data corresponding to an alternative prescription to said first program in a data format selected to be compatible with said first program.

Alternatively, the system may return control to the first program at the input stage, allowing the practitioner to input his chosen alternative prescription *ab initio*.

In a preferred embodiment of the present invention, the system is provided with a means of updating said database of prescriptions and possible alternatives, with data provided by a central source.

Advantageously, updates to said database are provided online, by means of electronic mail or the Internet, in which case said database may be updated either on a real-time basis, as new information becomes available to the central source, or periodically, for example, daily, hourly or every ten minutes.

Alternatively, said updates may be provided by CD-ROM, by floppy disc or by other suitable pre-programmed means according to available resources.

In another preferred embodiment of the present invention, the alternatives to the proposed prescription are provided to the practitioner along with further medical or cost information to explain or expand on the reasons for considering said alternatives.

Advantageously, means are provided to enable the practitioner to access more detailed information if he so wishes.

In this case, the more detailed information may be provided as part of said database or, optionally, may be held at said central source, in which case it may be provided by, for example, arranging access to restricted access websites.

In a further preferred embodiment of the present invention, the system is provided with means to supply, and optionally to print out, explanatory information adapted for the benefit of a patient for whom the prescription is intended.

In a fourth preferred embodiment of the present invention, said database of prescriptions and possible alternatives also comprises data on costs of the prescriptions and alternatives, and the system is provided with means to rank said alternatives on the basis of cost and to present said alternatives to the practitioner along with an indication of the costs involved.

Advantageously, the system may also be provided with means to calculate any savings made when the practitioner does choose an alternative prescription, and to record said savings in a further database, optionally linked to data such as, for example, but not exclusively, the prescription replaced, the alternative chosen and the practitioner making the choice.

An operator may then analyse said further database to establish and report on which alternatives or "switches" are being used most often, or which are producing the greatest cost savings, optionally compared with the savings expected when the alternatives were introduced. The system may transfer such data automatically to a central point, for example, the central source of data on alternative prescriptions, to enable such analysis to be carried out on combined data from several medical practices.

Further management information may be collected, analysed and reported preferably in a format not identifying individual practitioners, to aid efficient administration of this aspect of medical practice.

In a third aspect of the present invention, there is provided a computer program comprising program code means for initiating each of the steps of any one of the aspects described above, when the program is run on a computer.

Embodiments of the present invention will now be more particularly described, by way of example and with reference to the accompanying drawings, in which:-
**Figure 1** is a schematic representation of the components of an existing system for processing and printing out prescriptions; and
**Figure 2** is a schematic representation of the components of a system embodying the second aspect of the present invention, operating in conjunction with the system shown in Figure 1.

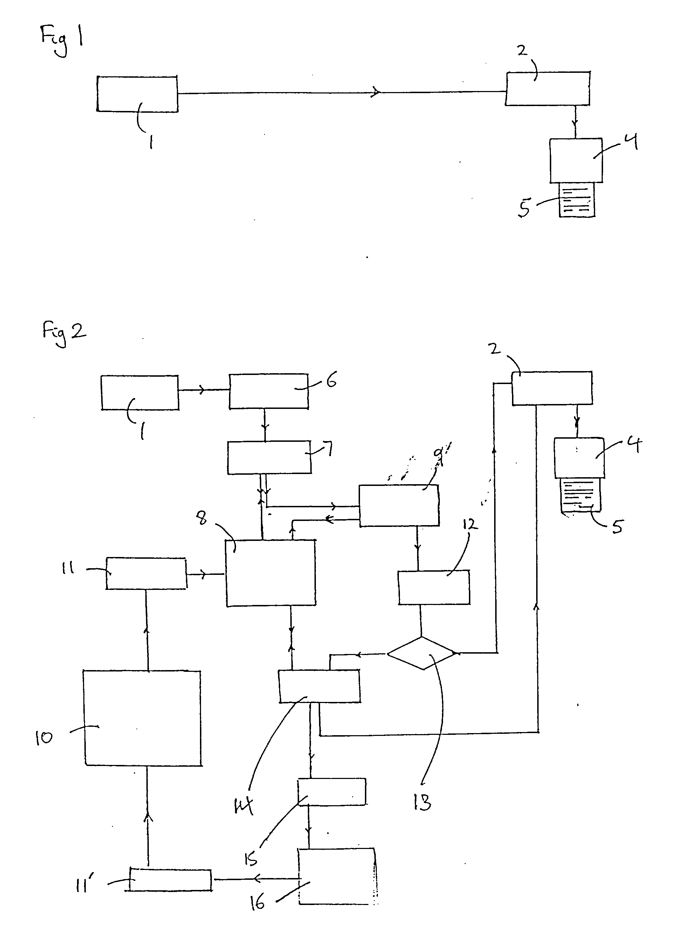

Referring now to the drawings and to Figure 1 in particular, in an existing system for processing and printing out prescriptions, a prescription for a patient is input by a medical practitioner at element 1. An element 2 transmits instructions to a printer 4 to print out a prescription form 5 in an appropriate layout for the practitioner to sign.

Referring now to Figure 2, a computer program embodying the second aspect of the present invention is running simultaneously with the system described above. A prescription is input by a medical practitioner at element 1, as before. A program element 6 may then be activated, or it may be running continuously "in the background", until it recognises a data input in the format appropriate to element 1, when it suspends operation of element 2 and routes said input instead to element 7. Said element 7, referring to database 8, identifies the proposed prescription that was input at element 1, and passes this information to element 9, which, also referring to database 8, generates alternative prescriptions to that proposed. Database 8 holds details of possible prescriptions and alternatives to said prescriptions with similar clinical effect. It is updated by communication with a central source of data 10, via a communications means 11, which may, for example, but not exclusively, take the form of an Internet connection or a CD-ROM data disc.

The alternative prescriptions generated by element 9 are presented to the practitioner by means 12 on a display screen. Means 12 has associated selection means 13, adapted to enable the practitioner to select one of the alternatives presented by 12, or to select the original proposed prescription input at element 1. In the latter case, the system returns control to element 2, which resumes operation using the original prescription input at element 1, as shown in Figure 1. If the practitioner selects an alternative prescription, that information is passed to element 14, which, referring to database 8, generates an input signal, in a data format adapted to be read by element 2, corresponding to the alternative prescription. This is then transmitted to element 2, which resumes operation continuing as if it had received said input signal, generated by element 14, directly from element 1. The alternative prescription is thus printed out, in the same manner as for the existing system.

As described above, the system may be provided with further details on the alternative prescriptions, held in database 8, which may additionally be presented by means 12, to aid the practitioner in this decision making. In such an embodiment, selection means 13 may additionally be so adapted as to enable the practitioner to request additional information from database 8, or optionally from the central source of data 10.

In embodiments of the present invention in which such further data is available, element 14 may also be adapted to transmit instructions directly to printer 4, to cause it to print out said further data, in a layout adapted for the use of the practitioner, or alternatively for the use of the patient in understanding the implications of the prescription.

In embodiments of the present invention in which cost data is provided, associated with the alternative prescriptions, initial presentation of this data to the practitioner may be accomplished by means of elements 9 and 12. In those embodiments where cost savings may be calculated and recorded, requisite data may be passed from element 14 to an element 15 adapted for the purpose, which is provided with a dedicated database 16 to store results for subsequent analysis. These results may be transmitted to the central source of data 10 via communications means 11¹.

In all embodiments of the present invention the element 9, which generates alternatives to the proposed prescription, may be adapted to return control directly to element 2, should no appropriate alternative prescriptions be generated. In this situation, the practitioner will notice no difference in operation, compared to the existing system shown in Figure 1.

As there are many existing computer programs and other systems which may carry out the functions shown in Figure 1, it is advantageous if a system embodying the present invention is provided with means to recognise with which such existing system it is being required to operate. It may then be so adapted as to select automatically the appropriate data format to monitor for, in order to trigger its operation, and to transmit back to the existing system when an alternative prescription is to be printed out.

It is preferable that the central source of data is administered by an official body such as the National Health Service, N I C E or an organ thereof, to ensure consistent and impartial handling of data on both clinical equivalence and cost effectiveness of alternative prescriptions. This also ensures that the accounting basis behind the cost data is the same as that used to administer the medical practitioners' prescribing budget.

A computer program embodying the first aspect of the present invention runs in a similar manner. A prescription for a patient is entered by a medical practitioner at element 1, but before he causes the prescription to be transmitted to element 2, he manually activates element 7. The practitioner enters the proposed prescription into element 7, which passes this information to element 9, which, referring to database 8, generates alternative prescriptions to that proposed. Database 8 holds details of possible prescriptions and alternatives, as described above, and may be updated by communication with the central source of data 10 via the communications means 11, also as described above.

The alternative prescriptions generated by element 9 are presented to the practitioner by means 12 on a display screen. Means 12 has associated selection means 13, adapted to enable the practitioner to select one of the alternatives presented by 12, or to select the original proposed prescription input at element 1. In the latter case, the program ceases operation and returns control to element 1, permitting the practitioner to complete processing of the prescription as shown in Figure 1.

If the practitioner chooses an alternative prescription, this information is passed to element 15, which records the "switch" made and any consequent cost savings in the dedicated database 16. As described above, the information collected in the database 16 may be transmitted to the central source of data 10 via communication means 11¹.

The practitioner may then transfer control back to element 1 and manually enter the alternative prescription in place of that initially proposed, completing processing of the alternative prescription as shown in Figure 1. The program embodying the invention may then be reset for use to check a subsequent separate proposed prescription.

## Claims

1. A system to supply a medical practitioner with possible alternatives to a proposed prescription, **characterised in that** it comprises means to compare said prescription to a database of prescriptions, means to select from said database the proposed prescription and any possible alternative thereto, means to present an alternative prescription to the practitioner, whereby the practitioner may choose between his proposed prescription and any alternative presented to him, means to permit the practitioner to enter a prescription selected from the proposed or an alternative prescription, and means to process and print out the selected prescription.

2. A system as claimed in claim 1, **characterised in that** it is further provided with means of updating said database of prescriptions and possible alternatives thereto.

3. A system as claimed in claim 2, **characterised in that** the means of updating said database are provided online, by means of electronic mail or the Internet, so that said database may be updated either on a real-time basis, as new information becomes available, or periodically.

4. A system as claimed in claim 2, **characterised in that** the means of updating comprises CD-ROM, floppy disc or by other suitable pre-programmed means.

5. A system as claimed in any one of the preceding claims, **characterised in that** means are provided to supply to the practitioner further medical or cost information regarding the alternatives.

6. A system as claimed in any one of the preceding claims, **characterised in that** said database of prescriptions also comprises data on costs of the prescriptions and of the alternatives, and the system is provided with means to rank said alternatives on the basis of cost and means to present said alternatives to the practitioner along with an indication of the costs involved.

7. A system as claimed in claim 6, **characterised by** further comprising means to calculate any savings made when the practitioner does choose an alternative prescription, and means to record said savings in a further database.

8. A method of supplying a medical practitioner with possible alternatives to a proposed prescription, which responds to an input of said prescription to a first computer program adapted to process and print out said prescription, **characterised in that** the method comprises the steps of:-
- interrupting operation of said first program;
- comparing said prescription to a database of prescriptions and possible alternative prescriptions thereto;
- presenting any prescriptions alternative to said input prescription to the practitioner;
- providing the practitioner with means to choose between said input prescription and any alternative presented to him; and
- resuming operation of said first program, either using original data corresponding to the input prescription or using substitute data supplied by the system corresponding to a selected alternative presented, according to the choice made by the practitioner.

9. A method as claimed in claimed in claim 8, **characterised in that** the method further comprises the step of updating said database of prescriptions and possible alternatives.

10. A method as claimed in either claim 8 or claim 9, **characterised by** the further step of providing computer program means adapted to initiate each of said steps when the program is run on a computer.
